# FASCICULE DE BREVET EUROPEEN

(11) **EP 0 605 669 B1**
(45) Date de publication et mention de la délivrance du brevet: **04.03.1998**
(21) Numéro de dépôt: 93909379.5
(22) Date de dépôt: 28.04.1993
(51) Int. Cl.: A61M 5/168, G05D 7/06

(54) **DISPOSITIF DE CONTROLE DE DEBIT POUR SYSTEMES DE PERFUSION**
DURCHFLUSSKONTROLLVORRICHTUNG FÜR INFUSIONSSYSTEME
FLOW CONTROL DEVICE FOR INFUSION SYSTEMS

(30) Priorité: 29.04.1992 FR 9205305
(43) Date de publication de la demande: 13.07.1994
(73) Titulaire: CHRONOTEC S.A.R.L., F-06600 Antibes (FR)
(72) Inventeur: HAUSER, Jean-Luc, F-06600 Antibes (FR); GUYOMAR, Daniel, F-06200 Nice (FR); SAUZADE, Jean, Denis, F-06130 Grasse (FR)
(74) Mandataire: Bonneau, Gérard
(86) Numéro de dépôt international: FR9300410
(87) Numéro de publication internationale: WO9321977

(56) Documents cités:
- EP-A- 0 205 381
- DE-A- 3 507 106
- GB-A- 1 593 495
- US-A- 4 142 553

## Description

### Domaine technique

La présente invention concerne un dispositif de contrôle de débit de la substance médicamenteuse dans une ligne de perfusion du type comprenant une vanne comportant un moyen d'obturation de la ligne de perfusion et un circuit de commande de la position du moyen d'obturation dans le but de contrôler le débit de la substance médicamenteuse à travers la vanne.

### Etat de la technique

Les traitements d'un certain nombre de maladies telles que le diabète, le cancer..., par chimiothérapie exigent que l'on pratique des perfusions de façon continue ou intermittente, dans le but d'administrer une ou plusieurs substances médicamenteuses au patient.

Les systèmes de perfusion peuvent être soit du type fixe, généralement en milieu hospitalier, où l'écoulement de la substance médicamenteuse se fait par gravitation, par exemple à partir de poches plastiques accrochées au-dessus du lit du patient, soit du type ambulatoire à l'aide d'une pompe que le patient porte sur lui et qui permet l'injection automatique de la substance médicamenteuse dans le corps du patient. Que ce soit l'un ou l'autre type de système de perfusion, il est parfois nécessaire d'améliorer l'efficacité et la sécurité du système en lui ajoutant un dispositif de contrôle du débit.

On trouve dans la technique antérieure différents moyens de contrôle du débit d'écoulement de la substance médicamenteuse. Ainsi le brevet US 4 623 331 décrit un système de perfusion du type poche suspendue dans lequel un signal d'alarme est produit lorsque le nombre de gouttes délivrées par la ligne de perfusion est incorrect. Ce n'est donc pas, à proprement parler un dispositif de contrôle de débit mais plutôt un simple dispositif de sécurité.

Le brevet US 4 616 801 décrit un système dans lequel un galet excentré vient comprimer la ligne de perfusion lors de sa rotation commandée par un moteur électrique. L'effort d'écrasement permet de moduler le débit d'écoulement. Il va de soi que la précision d'un tel système est très approximative.

Le brevet US 4 038 981, qui décrit les caractéristiques du préambule de la revendication 1, décrit une ligne de perfusion comportant une vanne électromagnétique à bille permettant de réguler le nombre de gouttes en fonction du temps, de façon à fixer le nombre de gouttes entre une valeur minimale et une valeur maximale. La commande électromagnétique de la vanne a seulement pour but de mettre la bille en position haute ou basse c'est-à-dire d'ouvrir ou de fermer la vanne. On voit donc que ce système ne permet pas un contrôle continu du débit de la substance médicamenteuse mais seulement le comptage du nombre de gouttes fournies sans aucune possibilité de contrôler le volume de chaque goutte.

### Exposé de l'invention

L'invention a donc pour but de réaliser un contrôle continu du débit d'écoulement de la substance médicamenteuse dans une ligne de perfusion.

L'objet de l'invention est donc un dispositif de contrôle du débit de la substance médicamenteuse dans une ligne de perfusion comprenant une vanne comportant un moyen d'obturation de la ligne de perfusion et un circuit de commande de la position du moyen d'obturation, la vanne comprenant une chambre ayant une première ouverture communiquant avec la partie amont de la ligne de perfusion et une seconde ouverture communiquant avec la partie aval de la ligne de perfusion, et le circuit de commande étant extérieur à ladite chambre. Un dispositif bilame se trouve à l'intérieur de la chambre en étant solidaire d'une des parois de la chambre et le moyen d'obturation est solidaire du dispositif bilame et de forme appropriée pour fermer en tout ou partie l'une des première et seconde ouvertures. Le circuit de commande est connecté au dispositif bilame pour contrôler une tension V1 ou deux tensions V1, V2 appliquées au dispositif bilame de manière à obtenir un contrôle continu et précis de la position du moyen d'obturation par rapport aux dites ouvertures et donc du débit de la substance médicamenteuse à travers la vanne et dans la ligne de perfusion.

Les revendications dépendantes spécifient des modes de réalisation préférentiels de l'invention.

### Brève description

L'invention sera mieux comprise à la lecture de la description qui suit faite en référence aux dessins dans lesquels :
la figure 1 représente une vue en coupe d'un mode de réalisation préféré du dispositif de contrôle selon l'invention,
la figure 2 représente une vue en coupe d'un premier type de bilame piézo-électrique pouvant être utilisé dans le dispositif de contrôle selon l'invention,
la figure 3 représente une vue en coupe d'un deuxième type de bilame piézo-électrique pouvant être utilisé dans le dispositif de contrôle selon l'invention.

### Description détaillée

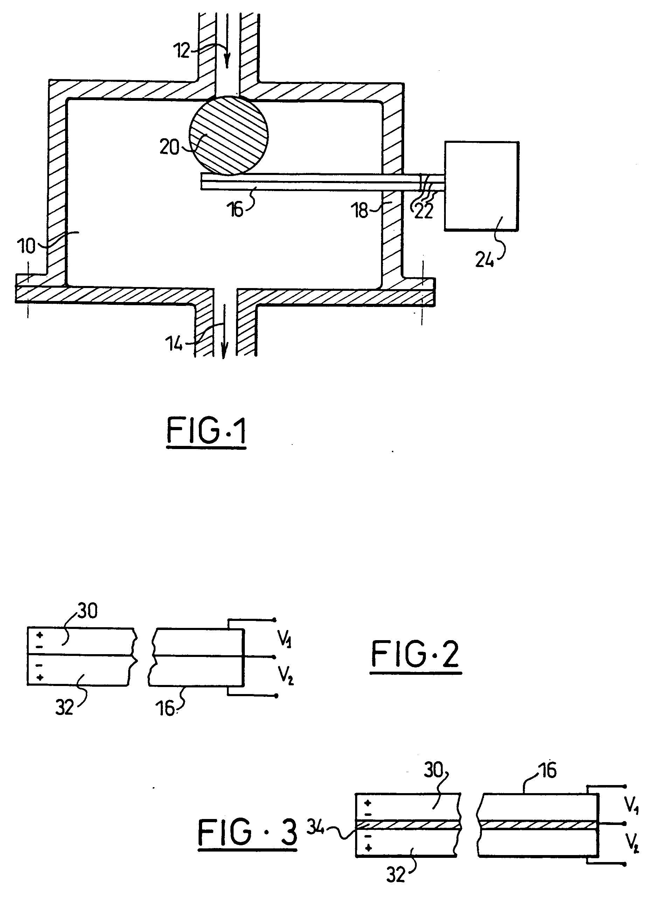

Code illustré sur la figure 1, la ligne de perfusion comporte une chambre 10 communiquant avec l'entrée de la ligne de perfusion par l'ouverture 12 et communiquant avec la sortie de la ligne de perfusion par l'ouverture 14. Un dispositif bilame 16 du type piézo-électrique est encastré dans une des parois 18 de la chambre 10. Une bille 20 est montée solidaire du bilame 16 de telle sorte que dans la position de repos du bilame 16, la bille vienne fermer l'ouverture d'entrée 12 de la ligne de perfusion.

Le dispositif bilame est connecté par une liaison à 3 fils 22 à un circuit de commande 24 qui applique des tensions à chacune des deux lames du bilame de façon à ce que la lame supérieure s'allonge pendant que la lame inférieure se rétracte, comme on va le voir plus en détail par la suite. Le bilame est forcé à fléchir entraînant la bille 20 vers le bas ce qui a pour effet de libérer l'ouverture d'entrée 12. Des tensions appliquées aux lignes d'entrée 22 par le circuit de commande 24 dépend le plus ou moins grand fléchissement du bilame 16, donc un débit de la substance médicamenteuse pouvant être réglé avec une grande précision.

Bien que dans le mode de réalisation préféré de l'invention le moyen d'obturation de l'ouverture 12 soit une bille 20, il est clair pour l'homme du métier que ce moyen d'obturation pourrait être de toute autre forme appropriée telle qu'un cône, un disque ou même une surface de forme dissymétrique. Cependant, il est clair que la bille présente un avantage sur d'autres moyens d'obturation en ce sens qu'elle s'adapte facilement à son siège et permet en outre une bonne précision principalement pour les faibles débits, ce qui n'est pas le cas pour toute forme du moyen d'obturation.

Il est clair également que le contrôle du débit pourrait se faire par obturation de l'ouverture de sortie 14 plutôt que par obturation de l'ouverture d'entrée 12. Dans ce cas le bilame 16 devra être adapté pour fléchir vers le haut, dans le mode de réalisation de la figure 1, pour augmenter le débit de la substance médicamenteuse. Enfin, il est possible de prévoir qu'en position de repos, la bille 20 ou tout autre moyen d'obturation approprié, ne bloqué pas entièrement l'ouverture 12 (ou 14) mais laisse une ouverture partielle de sorte que le débit ne soit pas réduit à zéro en cas de défaillance de l'application des tensions par le, circuit de commande 24.

La figure 2 représente un mode de réalisation du dispositif bilame piézo-électrique 16 de l'invention. Le bilame 16 est constitué de deux lames 30 et 32 d'un matériau piézo-électrique, polarisées. Les deux lames sont métallisées sur leurs deux faces extérieures, ainsi que leur face commune, de sorte que l'application d'une tension V1, à la lame 30 et V2 à la lame 32, soit uniforme pour toute la lame. Par combinaison de deux tensions appropriées V1 et V2 on peut obtenir un allongement de la lame 30 et une contraction de la lame 32 de sorte que le dispositif bilame se courbe vers le bas (ou vers le haut). Ainsi, des tensions appropriées pour V1 et V2 sont comprises entre 0 et 20 volts. L'une des tensions peut par exemple varier en fonction du débit à obtenir, l'autre tension étant fixée à 20 volts; ou bien chacune des tensions peut varier de 0 à 20 volts, seule la différence entre les deux tensions étant utilisée pour commander le débit.

Le dispositif bilame se trouvant dans le flot du liquide perfusé, il doit être en un matériau permettant de conserver une grande stabilité et une grande fidélité, c'est à dire que sa réponse doit être toujours la même quel que soit le liquide perfusé, et ceci dans une gamme de température allant de 10°C à 25°C. En outre, et c'est une des caractéristiques de l'invention, le temps de réponse du dispositif bilame doit être le plus petit possible. Ces caractéristiques peuvent être obtenues en utilisant de préférence une céramique piézo-électrique ou tout autre matériau ayant des propriétés équivalentes.

Bien entendu, le dispositif de contrôle de débit selon l'invention doit être étalonné avant toute utilisation en milieu de perfusion. Cet étalonnage peut être réalisé avec de l'eau à une température comprise entre 15 et 20°C.

Un deuxième mode de réalisation du dispositif bilame 16 est représenté sur la figure 3. Dans ce mode de réalisation, une feuille métallique 34 est encastrée entre les deux lames 30 et 32. Cette feuille métallique est bien entendu, plus épaisse qu'une simple métallisation comme illustré sur la figure 2. L'avantage d'utiliser une feuille métallique entre les deux lames de matériau piézo-électrique est de renforcer la rigidité du bilame et d'améliorer la précision du contrôle de débit. Un autre mode de réalisation possible (non représenté) consisterait à n'utiliser qu'une lame active soumise à une tension variable, l'autre étant passive, par exemple une simple lame métallique reliée à la masse.

Le circuit de commande 24 chargé d'appliquer les tensions de contrôle V1 et V2 au bilame recueille en fait des courants en provenance des lames dont l'intensité permet de contrôler l'état de contrainte dans le bilame. Les tensions V1 et V2 pourront donc être déterminées en fonction des courants recueillis sur les fils 22, et le circuit 24 peut donc être un circuit du type dans lequel la génération des tensions V1 et V2 appliquées au bilame est asservie aux courants mesurés à la sortie des fils 22. Ce type de circuit est à la portée de l'homme du métier et n'est donc pas représenté sur les dessins.

En outre, le circuit de commande peut être réalisé en tant que circuit autonome comportant des moyens d'entrée permettant une programmation du débit de la substance médicamenteuse souhaité, ou bien être relié à une commande centralisée telle qu'un dispositif de pompe ambulatoire programmable, la programmation des conditions de débit étant alors effectuée à partir du dispositif de pompe programmable. De même, le circuit de commande 24 peut être relié à un capteur de pression situé en amont dans la ligne de perfusion de façon à régler le débit en fonction de la pression dans la ligne de perfusion.

## Revendications

1. Dispositif de contrôle du débit de la substance médicamenteuse dans une ligne de perfusion comprenant une vanne comportant un moyen d'obturation (20) de la ligne de perfusion et un circuit de commande (24) de la position du moyen d'obturation, ladite vanne comprenant une chambre (10) ayant une première ouverture (12) communiquant avec la partie amont de la ligne de perfusion et une seconde ouverture (14) communiquant avec la partie aval de la ligne de perfusion, et ledit circuit de commande étant extérieur à ladite chambre ;
ledit dispositif étant caractérisé par un dispositif bilame (16) se trouvant à l'intérieur de ladite chambre et solidaire d'une paroi (18) de ladite chambre, ledit moyen d'obturation étant solidaire dudit dispositif bilame et de forme appropriée pour fermer en tout ou partie l'une desdites première et seconde ouvertures, ledit circuit de commande étant connecté audit dispositif bilame pour contrôler une tension V1 ou deux tensions V1, V2 appliquées audit dispositif bilame de manière à obtenir un contrôle continu et précis de la position dudit moyen d'obturation par rapport à ladite ouverture et donc du débit de la substance médicamenteuse à travers la vanne et dans la ligne de perfusion.

2. Dispositif selon la revendication 1, caractérisé en ce que ledit circuit de commande (24) établit la tension V1 ou les tensions (V1, V2) appliquées audit dispositif bilame (16) en fonction des courants circulant dans ledit dispositif bilame.

3. Dispositif selon la revendication 1 ou 2, caractérisé en ce que ledit moyen d'obturation (20) ferme en tout ou partie ladite première ouverture (12) communiquant avec la partie amont de la ligne de perfusion.

4. Dispositif selon l'une quelconque des revendications précédentes, caractérisé en ce que ledit dispositif bilame est composé de deux lames (30, 32) formées d'un matériau piézo-électrique tel que de la céramique.

5. Dispositif selon la revendication 4, caractérisé en ce que ledit dispositif bilame (16) comprend en outre une feuille de métal (34) encastrée entre lesdites lames de matériau piézo-électrique (30, 32).

6. Dispositif selon l'une quelconque des revendications précédentes, caractérisé en ce que ledit moyen d'obturation (20) est une bille.

## Claims

1. Device controlling the flow of therapeutic solution in a infusion line comprising a valve including a means (20) for closing the infusion line and a control circuit (24) for the position of the closing means ; said valve comprising a chamber (10) with a first aperture (12) communicating with the upstream part of the infusion line and a second aperture (14) communicating with the downstream part of the infusion line, and said control circuit being outside said chamber,
said device being characterized by a bimetal device (16) located inside said chamber and attached to one side (18) of said chamber, said closing means being attached to said bimetal device and having an appropriate form to close one of said first and second apertures in whole or in part, said control circuit being connected to said bimetal device to control a voltage V1 or two voltages V1, V2 applied to said bimetal device in order to obtain constant accurate control of the position of said closing means with respect to said aperture and thus the flow of therapeutic solution through the valve and in the infusion line.

2. Device according to claim 1, characterized in that said control circuit (24) establishes voltage V1 or voltages (V1, V2) applied to said bimetal device (16) in terms of the currents flowing in the bimetal device.

3. Device according to claims 1 or 2, characterized in that said closing means (20) closes in part or in whole said first aperture (12) communicating with the upstream part of the infusion line.

4. Device according to any one of the preceding claims, characterized in that said bimetal device is composed of two blades (30, 32) made of piezoelectric material, such as ceramic.

5. Device according to claim 4, characterized in that said bimetal device (16) further includes a metal strip set between said blades made of piezoelectric material (30, 32).

6. Device according to any one of the preceding claims, characterized in that said closing means (20) is a ball.

## Patentansprüche

1. Vorrichtung zur Steuerung der Durchflußmenge der pharmazeutischen Substanz in einer Infusionsleitung, mit einem Ventil, das eine Einrichtung (20) zum Verschließen der Infusionsleitung enthält, und mit einem Steuerkreis (24) für die Position der Verschlußeinrichtung, wobei das Ventil eine Kammer (10) mit einer ersten Öffnung (12), die mit dem stromaufwärtigen Teil der Infusionsleitung (14) verbunden ist, sowie eine zweiten Öffnung aufweist, die mit dem stromabwärtigen Teil der Infusionsleitung verbunden ist, und wobei der Steuerkreis sich außerhalb der Kammer befindet;
gekennzeichnet durch eine Bimetalleinrichtung (16), die sich innerhalb der Kammer befindet und mit einer Wand (18) der Kammer fest verbunden ist, wobei die Verschlußeinrichtung mit der Bimetalleinrichtung fest verbunden ist und eine geeignete Form hat, um ganz oder teilweise eine der ersten und zweiten Öffnungen zu schließen, und wobei der Steuerkreis an die Bimetalleinrichtung angeschlossen ist, um eine Spannung V1 oder zwei Spannungen V1, V2 zu steuern, die an der Bimetalleinrichtung anliegen, so daß eine kontinuierliche und präzise Steuerung der Position der Verschlußeinrichtung in Bezug auf die Öffnung und folglich der Durchflußmenge der pharmazeutischen Substanz durch das Ventil hindurch und in die Infusionsleitung erhalten wird.

2. Vorrichtung nach Anspruch 1, dadurch gekennzeichnet, daß der Steuerkreis (24) die Spannung V1 oder die Spannungen (V1, V2) aufbaut, die an der Bimetalleinrichtung (16) als Funktion der Ströme anliegt bzw. anliegen, die in der Bimetalleinrichtung fließen.

3. Vorrichtung nach Anspruch 1 oder 2, dadurch gekennzeichnet, daß die Verschlußeinrichtung (20) die erste Öffnung (12) ganz oder teilweise schließt, die mit dem stromaufwärtigen Teil der Infusionsleitung verbunden ist.

4. Vorrichtung nach einem der vorhergehenden Ansprüche, dadurch gekennzeichnet, daß die Bimetalleinrichtung aus zwei Streifen (30, 32) zusammengesetzt ist, die aus einem piezoelektrischen Material wie Keramik gebildet sind.

5. Vorrichtung nach Anspruch 4, dadurch gekennzeichnet, daß die Bimetalleinrichtung (16) ferner eine Metallfolie aufweist, die zwischen den Streifen (30, 32) aus piezoelektrischem Material eingebaut ist.

6. Vorrichtung nach einem der vorhergehenden Ansprüche, dadurch gekennzeichnet, daß die Verschlußeinrichtung (20) eine Kugel ist.
